# EUROPEAN PATENT APPLICATION

(11) **EP 4 116 330 A1**
(43) Date of publication of application: **11.01.2023**
(21) Application number: 21184105.1
(22) Date of filing: 06.07.2021
(51) Int. Cl.: C07K 16/30, C07K 16/28, C07K 16/46, A61P 35/00, A61K 39/00

(54) **MULTISPECIFIC ANTIBODIES WITH MONOVALENT BINDING TO TUMOR-ASSOCIATED ANTIGENS CAUSING NO OR REDUCED NERVE PAIN IN THE TREATMENT OF CANCER**

(30) Priority: 05.07.2021 EP 21183796
(71) Applicant: Trion Research GmbH, 82152 Martinsried (DE)
(72) Inventor: Lindhofer, Horst, 80639 München (DE); Ruf, Peter, 82296 Schöngeising (DE)
(74) Representative: Isarpatent

(57) **Abstract**

The present invention relates to an intact whole IgG bispecific antibody, comprising the following properties: a) binding to a T cell; b) binding to a tumor-associated antigen on a tumor cell; c) no or only marginal complement dependent cytotoxicity at antibody concentrations <500ng/ml resulting in the avoidance of nerve pain, and for use in treatment of neuroblastoma or sarcoma, melanoma, glioma, small cell lung cancer, triple negative breast cancer.

## Description

### Technical field

The present invention relates to a multispecific antibody binding to tumor-associated antigens causing no or reduced nerve pain in the treatment of cancer.

### Background

The complement system plays a critical role in the interaction between the innate and adaptive immune response, whereby to achieve the effect of damaging cancer cells. There are membrane-bound complement regulatory proteins (mCRPs), including for example CD55, CD46, and CD59, which are expressed in the body to achieve the suppression of over-activation of the complement system. However, on the other hand, the mCRPs can also over-regulate the complement system and therefore cannot anymore destroy the cancerous cells. It has been investigated that in many cancer types mCRPs can serve as a biomarker for malignant transformation, which therefore should be taken into account in treatment of cancer.

The tumor-associated ganglioside GD2 is an attractive target for immunotherapy. While its expression in normal tissue is restricted to the central nervous system and peripheral nerves [1,2] it is strongly detectable on neuroblastoma (NB) and on most melanoma lesions [3,4]. Additionally, it is found on sarcoma, glioma and in approximately 50%-100% of small cell lung cancers (SCLC) where it is associated with enhanced cell proliferation and invasive activity [5-7]. Due to its limited expression on normal tissue, the GD2 disialogangloside expressed on neuroblastoma cells is in principle an excellent candidate for mAb therapy.

Many antigens overexpressed on neuroblastoma are often present at lower levels in peripheral nerves and/or on other neural tissue [8-10], so an important consideration in the development of anti-GD2 antibodies is the potential for off-tumor, on-target toxicity. Anti-GD2 monoclonal antibodies cause pain requiring continuous infusion of narcotics for analgesia [11-14] due to their interaction with peripheral nerves and possible engagement of the complement system [15].

The approved antibody Dinutuximab (17.5 mg/m2/day), when administered in combination with GM-CSF, IL-2, and RA, has a significant, relatively manageable, toxicity profile. In the pivotal phase III trial (COG ANBL0032) that compared dinutuximab 17.5 mg/m2/day, GM-CSF, IL-2 (3 x 106 or 4.5 x 106 IU/m2/ day), and RA with standard therapy (RA), more than 90% of the 137 patients receiving dinutuximab experienced a treatment-related Adverse Event [16].

Grade 3 or 4 toxicities that occurred more often in patients receiving dinutuximab as compared with those in the standard-therapy group included pain (52% vs 6%), hypersensitivity reactions (25% vs 1%), capillary leak syndrome (23% vs 0%), and hypotension (16% vs 0%) (12,26). The pain associated with dinutuximab administration was often neuropathic, most often affecting abdominal sites, and occurred most frequently with cycle 1, with a trend toward reduced frequency during subsequent cycles.

Thus, there is an increasing desire of reducing or even eliminating the nerve pain during the treatment of tumor or cancer.

### Detailed Description of the Invention and Preferred Embodiments

The following discussion is included for purposes of describing the present invention and illustrating preferred embodiments thereof.

Unless otherwise explained, all technical and scientific terms used herein have the same meaning as commonly understood by one of ordinary skill in the art to which this disclosure belongs. The singular terms "a," "an," and "the" include plural referents unless context clearly indicates otherwise. Similarly, the word "or" is intended to include "and" unless the context clearly indicates otherwise. Although methods and materials similar or equivalent to those described herein can be used in the practice or testing of the present disclosure, suitable methods and materials are described below. The term "comprises" means "includes." All publications, patent applications, patents, and other references mentioned herein are incorporated by reference in their entirety. In case of conflict, the present specification, including explanations of terms, will control. In addition, the materials, methods, and examples are illustrative only and not intended to be limiting.

The present invention concerns:
(1). An intact whole IgG bispecific antibody, comprising the following properties:
   a) binding to a T cell;
   b) binding to a tumor-associated antigen on a tumor cell;
   c) no or only marginal complement dependent cytotoxicity at antibody concentrations <500ng/ml resulting in the avoidance of nerve pain. Preferably, the concentration refers to the concentration of the antibody in the blood of the subject.
(2). Preferably, the intact bispecific antibody of (1), wherein the tumor-associated antigen is selected from a group consisting of tumor associated sphingolipids and sphingoid molecules, gangliosides or glucosphingolipids like e.g. GD2, GD3, GM1, GM2, GM3, fucosyl-GM1, globo-H, S1P, Cer, and Gg3, preferably the tumor-associated antigen is the neuroblastoma-associated antigen GD2.
(3). Preferably, the intact bispecific antibody of (1) or (2), wherein said intact bispecific antibody binds to the T cell through a T cell surface antigen, and wherein the T cell surface antigen is selected from a group consisting of CD2, CD3, CD4, CD8, CD28, CD40L and CD44, preferably the T cell surface antigen is CD3.
(4). Preferably, the intact bispecific antibody of any one of (1) to (3), wherein said antibody comprises an Fc- portion where the complement binding amino acids within the Fc-portion are modified by one of substitution, insertion and deletion.
(5). A pharmaceutical composition comprising the intact bispecific antibody of any one of (1) to (4).
(6). Preferably, the pharmaceutical composition of (5), further comprising a pharmaceutically suitable carrier.
(7). Preferably, the intact bispecific antibody of any one of (1) to (6) for use as a medicament.
(8). Preferably, the intact bispecific antibody of any one of (1) to (4) for use in treatment of neuroblastoma or sarcoma, melanoma, glioma, small cell lung cancer, breast cancer, triple negative breast cancer, more preferably neuroblastoma, breast cancer or triple negative breast cancer.
(9). Preferably, the intact bispecific antibody for use of (7) or (8), wherein the antibody binds to cancer stem cell or cancer stem-like cells.
(10). Preferably, the intact bispecific antibody for use of any one of (7) to (9), wherein the antibody binds to breast cancer stem cell or breast cancer stem-like cells.
(11). Preferably, the intact bispecific antibody for use of any one of (7) to (10), wherein said antibody binds to GD2 expressed on the surface of tumor, cancer, cancer stem cell, or cancer stem-like cells.
(12). Preferably, the intact bispecific antibody for use of any one of (7) to (11), wherein said bispecific antibody is an anti-GD2 x anti-CD3 antibody.
(13). Preferably, the intact bispecific antibody for use of any one of (7) to (12), wherein said intact bispecific antibody is administered in an amount of 10 µg/m² body surface to 10 mg/m² body surface for one administration.
(14). Preferably, the intact bispecific antibody for use of any one of (7) to (13), wherein said bispecific antibody is administered for several times
(15). Preferably, the intact bispecific antibody for use of any one of (7) to (14), wherein said bispecific antibody is administered through intravenous infusion, intraperitoneal infusion, intramuscular injection, subcutaneous injection, or direct injection to the tumor.

Preferably, above-disclosed intact bispecific antibody is a trifunctional bispecific antibody.

Preferably, the above-disclosed intact bispecific antibody may be monovalent, bivalent, trivalent, tetravalent or multivalent.

Preferably, the above-disclosed intact bispecific antibody may be monovalent or bivalent. Said monovalent binding property may be connected with a better effect in respect of no or only marginal complement dependent cytotoxicity at antibody concentrations <100ng/ml, more preferably at antibody concentrations <500ng/ml, and with an improved avoidance of nerve pain. Bivalent antibody displaying the similar binding affinity to the tumor-associated antigen with said monovalent antibody can also be connected with above-stated improved desired effects.

In the present invention, the term "tumor cell" refers to any cell which can divide relentlessly and form solid tumors or flood the blood with abnormal cells. Preferably, in the present invention, the tumor cells can be from epithelial, haematological or neuroectodermal tumors.

Examples of tumors included in the present invention (but not limited thereto) comprise sarcomas and carcinomas, including fibrosarcoma, myxosarcoma, liposarcoma, chondrosarcoma, osteogenic sarcoma, and other sarcomas, synovioma, mesothelioma, Ewing's tumor, leiomyosarcoma, rhabdomyosarcoma, colon carcinoma, lymphoid malignancy, pancreatic cancer, breast cancer (including basal breast carcinoma, ductal carcinoma and lobular breast carcinoma as well as triple negative breast cancer and GD2 expressing breast cancer stem-like cells), lung cancers, ovarian cancer, prostate cancer, hepatocellular carcinoma, squamous cell carcinoma, basal cell carcinoma, adenocarcinoma, sweat gland carcinoma, medullary thyroid carcinoma, papillary thyroid carcinoma, pheochromocytomas sebaceous gland carcinoma, papillary carcinoma, papillary adenocarcinomas, medullary carcinoma, bronchogenic carcinoma, renal cell carcinoma, hepatoma, bile duct carcinoma, choriocarcinoma, Wilms' tumor, cervical cancer, testicular tumor, seminoma, bladder carcinoma, and CNS tumors (such as a glioma, astrocytoma, medulloblastoma, craniopharyrgioma, ependymoma, pinealoma, hemangioblastoma, acoustic neuroma, oligodendroglioma, meningioma, melanoma, neuroblastoma and retinoblastoma). Further examples include epithelial tumors, hematological tumor, GD2 expressing cancer stem cells and neuroectodermal tumors.

Preferably, the tumor in the present invention is neuroblastoma.

Preferably, in the present invention, said tumor cells are from epithelial, hematological, GD2 expressing cancer stem cells or neuroectodermal tumors.

In the present invention, the "immune cells" include T cells and Fc-receptor positive cells. In the present invention, the "Fc-receptor cell" refers to cells with Fc-receptor present on the cell surface. Preferably, the "Fc-receptor positive cell" refer to one or more of monocyte, macrophage, dendritic cell, natural killer cell, neutrophil and eosinophilic cell.

In the present invention, said intact bispecific antibody may be capable of forming a 3-dimensional network of said antibody and said tumor cells. In a further particularly preferred embodiment of the present invention, said antibody is also capable of binding to more than one tumor cells and/or to immune cells in order to form a three-dimensional network of antibodies and tumor cells and optionally immune cells and further optionally additional tumor cells.

The present invention therefore preferably focuses on the depletion of tumor cells carrying tumor-associated antigens by destruction of said tumor cells by antibody-specific interactions and immunological effects involving T cells and/or Fc receptor positive immune cells. The present invention can gain benefit preferably on the antibodies' capacity of being capable in crosslinking antigens which are in the present case located on tumor cells or fragments thereof.

Preferably, the presently disclosed antibody displays a low to intermediate affinity to the tumor-associated antigen, more preferably in a range of K_{D} = 10⁻⁵ - 10⁻⁸ M. This preferred embodiment can be connected with a better effect in respect of no or only marginal complement dependent cytotoxicity at antibody concentrations <100ng/ml, more preferably at antibody concentrations <500ng/ml, and with an improved avoidance of nerve pain.

The present invention is herein described and claimed with respect to intact bispecific antibodies within the limitations of the present invention, and exemplified with respect to the bispecific antibody anti-CD3 x anti-GD2 (EKTOMUN). Said anti-CD3 x anti-GD2 bispecific antibody is directed against tumor-associated antigen GD2, and is additionally binding to CD3, a T cell surface antigen and to Fc-receptor positive cells by its Fc-portion.

The present invention is herein described and claimed with respect to intact bispecific antibodies within the limitations of the present invention, and exemplified with respect to the bispecific antibody anti-CD3 x anti-EpCAM (Catumaxomab). Said anti-CD3 x anti-EpCAM bispecific antibody is directed against tumor-associated antigen EpCAM, and is additionally binding to CD3, a T cell surface antigen and to Fc-receptor positive cells by its Fc-portion.

The specific embodiments described in the examples have to be understood as exemplary embodiments which provide evidence for the feasibility of the present invention. Having provided this evidence, the person skilled in the art will inevitably have the possibility to expand the concept to other tumors and other antibodies as far as covered by the present invention which are able to interact with said tumor cells and optionally said immune cells in order to provide for a three-dimensional network.

The antibodies of the present invention are bispecific antibodies. However, if the tri-, tetra- and multispecific antibodies also exhibit the same properties or effects, said bispecific antibody can also refer to a tri-, tetra- and multispecific antibody as used herein.

The antibodies of the present invention are preferably selected from trifunctional antibodies. The trifunctional antibody disclosed below refers to trifunctional bispecific antibody. However, if the tri-, tetra- and multispecific antibodies also exhibit the same properties or effects, said trifunctional antibody can also refer to a trifunctional tri-, tetra- and multispecific antibody as used herein.

Generally, a bispecific antibody is defined as an antibody capable of binding to two different types of antigens preferably via its variable region; a trispecific antibody is characterized by binding to three different types of antigens preferably via its variable region; a tetraspecific antibody is characterized by binding to four different types of antigens preferably via its variable region while a multispecific antibody is defined as being capable of binding multiple different types of antigens preferably via its variable region. As one specific example, the trifunctional bispecific antibody anti-CD3 x anti-EpCAM is defined by binding to the tumor-associated antigen EpCAM on the one hand and to the T cell surface antigen CD3 on the other hand as well as with accessory cells by its Fc part. As another specific example, the trifunctional bispecific antibody anti-CD3 x anti-GD2 is defined by binding to the tumor-associated antigen GD2 on the one hand and to the T cell surface antigen CD3 on the other hand as well as with accessory cells by its Fc part.

Generally, the bi-, tri-, tetra- and multispecific antibodies described above may be monovalent, bivalent, trivalent, tetravalent or multivalent.

An antibody with a monovalent binding property is defined as an antibody which is capable of binding to one tumor-associated antigen. A bivalent monoclonal antibody is defined as an antibody which is capable of binding to two tumor-associated antigens or one tumor-associated antigen and one immune cell-associated antigen. A trivalent monoclonal antibody is defined as an antibody which is capable of binding to three different tumor-associated antigens or two tumor-associated antigens and one immune cell-associated antigen or one tumor-associated antigen and two immune cell-associated antigens. A tetravalent monoclonal antibody is defined as an antibody which is capable of binding to four different tumor-associated antigens or two different tumor-associated antigens - each having two identical antigen binding arms - or two/three tumor-associated antigens and one immune cell-associated antigen or two tumor-associated antigens and two immune cell-associated antigens. A multivalent monoclonal antibody is defined as an antibody which is capable of binding to one or more tumor-associated antigens and/or one or more immune cell associated antigen. The term "binding to a tumor-associated antigen" is defined as binding to an epitope of said tumor-associated antigen on a tumor cell.

General description of bifunctional or trifunctional antibodies are described by Kontermann RE (ed.), Springer Heidelberg Dordrecht London New York, pp. 1-28 (2011) having bispecific or trispecific (trifunctional formats with bivalent, trivalent and tetravalent) binding properties to one tumor-associated antigen and to one or more surface antigens of leukocytes (i.e. cells of the immune system) are of importance for this patent application.
- Bispecific antibody formats with bivalent antigen binding features: e.g. scFv (e.g. BiTE class), Db, scDb, dsDb, DART, dAb₂/VHH₂, knob-into-holes derivates, SEED-IgG, heteroFc-scfv, Fab-scFv, CrossMabs
- Bi- (tri-) specific antibody formats with trivalent antigen binding features: e.g. triple body, DNL-F(ab)₃, scFv₂-_{CH1/CL}, dAb₃, Fab-scFv₂, IgG-scFab
- Bi- (tri-) specific antibody formats with tetravalent antigen binding features: e.g. IgG-scFv, scFv-IgG, scFv-Fc, F(ab')₂-scFv₂, sDb-Fc, scDb-C_{H}3, Db-Fc, scFv₂-H/L, DVD-Ig, tandAb, scFv-dhlx-scFv, dAb₂-IgG, two-in-one mAb, mAb², dAb-IgG, dAb-Fc-dAb.

Additional antibodies to be used according to the invention are described in the following references:
Müller D and RE Kontermann. In: Bispecific Antibodies. Kontermann RE (ed.), Springer Heidelberg Dordrecht London New York, pp. 83-100 (2011)

### scFv (BiTE)

Baeuerle PA, Zugmaier G and D Rüttinger. In: Bispecific Antibodies. Kontermann RE (ed.), Springer Heidelberg Dordrecht London New York, pp. 273-288 (2011)

### DVD-Ig

Tarcsa E, Fraunhofer W, Ghayur T, Salfeld J and J Gu. In: Bispecific Antibodies. Kontermann RE (ed.), Springer Heidelberg Dordrecht London New York, pp. 171-186 (2011)

### DNL-derivatives

Chang C-H, Rossi EA, Sharkey RM, DM Goldenberg. In: Bispecific Antibodies. Kontermann RE (ed.), Springer Heidelberg Dordrecht London New York, pp. 199-216 (2011)

### Two-in-one antibodies

Koeing P and G Fuh. In: Bispecific Antibodies. Kontermann RE (ed.), Springer Heidelberg Dordrecht London New York, pp. 187-198 (2011)

### CrossMabs

Schaefer et al. Proc. Natl. Acad. Sci. USA 108: 11187 (2011)

In the present invention, at least one bispecific antibody, preferably trifunctional bispecific antibody is used. Preferably, two or more said bispecific antibodies with different specificities can be combined for mediating the associates of tumor cells and immune cells.

Preferably, the bispecific antibody in the present invention is for use in treatment of cancer, wherein said antibody binds to cancer stem cell or cancer stem-like cells. In this regard, cancer stem cell refers to cancer cells which exhibit characteristics associated with normal stem cells, specifically the ability to give rise to all cell types found in a particular cancer sample. In addition, cancer stem-like cell refers to a subpopulation of tumor or cancer cells with elevated tumor-initiating or cancer-initiating potential.

Preferably, the bispecific antibody in the present invention is for use in treatment of neuroblastoma, breast cancer or triple negative breast cancer. In this regard, triple negative breast cancer refers to cancer that tests negative for estrogen receptors, progesterone receptors, and overexpressed HER2 protein.

Preferably, the bispecific antibody in the present invention is for use in treatment of breast cancer or triple negative breast cancer, wherein said antibody binds to breast cancer stem cell or cancer stem-like cells.

Preferably, GD2 is expressed in the above cancer stem cells or cancer stem-like cells.

Preferably, GD2 is expressed in the above breast cancer stem cells or breast cancer stem-like cells.

Preferably, the bispecific antibody in the present invention is used in in an amount of 10 µg/m² body surface to 10 mg/m² body surface for one administration, 10 µg/m² body surface to 800 µg/m² body surface for one administration, 10 µg/m² body surface to 500 µg /m² body surface for one administration, 10 µg/m² body surface to 200 µg /m² body surface for one administration, 10 µg/m² body surface to 100 µg /m² body surface for one administration, 10 µg/m² body surface to 50 µg /m² body surface for one administration, 800 µg/m² body surface to 1 mg /m² body surface for one administration, 1 mg/m² body surface to 2 mg /m² body surface for one administration, 2 mg/m² body surface to 4 mg /m² body surface for one administration, 4 mg/m² body surface to 6 mg /m² body surface for one administration, 6 mg/m² body surface to 8 mg /m² body surface for one administration, or 8 mg/m² body surface to 10 mg /m² body surface for one administration.

In this present invention, by using said bispecific antibody in the above ranges, an even better anti-cancer effect can be achieved.

In this present invention, by using said bispecific antibody in the above ranges, a better effect in respect of no or only marginal complement dependent cytotoxicity at antibody concentrations <100ng/ml, more preferably at antibody concentrations <500ng/ml, can be achieved and an improved avoidance of nerve pain can be achieved.

Preferably, the antibody concentration in the blood of the patient should be controlled in a range of <100ng/ml, more preferably in a range of <500ng/ml.

Preferably, the antibody concentration in the blood of the patient should be controlled in a range of 40-80 ng/ml, 40-120 ng/ml, 40-160 ng/ml, 40-200 ng/ml, 40-240 ng/ml, 80-240 ng/ml, 120 ng/ml to 240 ng/ml, 160 ng/ml to 240 ng/ml, 200 ng/ml to 240 ng/ml, 80 ng/ml to 200 ng/ml, or 120 ng/ml to 160 ng/ml. With this low amount of antibody used and the antibody concentrations measured in the patient's blood, a reduced amount of antibody may bind to the target cells to initiate the complement cascade, e.g. through binding C1q to the cell-bound antibody.

In this present invention, by using said bispecific antibody in the above ranges, an even better anti-cancer effect can be achieved.

In this present invention, by using said bispecific antibody in the above ranges, a better effect in respect of no or only marginal complement dependent cytotoxicity at antibody concentrations <100ng/ml, more preferably at antibody concentrations <500ng/ml, can be achieved and an improved avoidance of nerve pain can be achieved. This may be attributed to the fact that said ranges would result that reduced antibodies are bound on target cells, whereby the complement cascade would not be initiated.

Preferably, the presently disclosed antibody comprises an engineered Fc- portion where the complement binding amino acids within the Fc-portion are deleted, which can be connected with a better effect in respect of no or only marginal complement dependent cytotoxicity at antibody concentrations <1000ng/ml, more preferably at antibody concentrations <500ng/ml, and with an improved avoidance of nerve pain.

The bispecific antibody according to the present invention may bind to a T cell via a T cell surface antigen selected from a group consisting of CD2, CD3, CD4, CD8, CD28, CD40L and CD44. It means that the antibody for use according to the present invention preferably comprises a paratope which can recognize and bind to an epitope of a T cell surface antigen selected from the group consisting of CD2, CD3, CD4, CD8, CD28, CD40L and CD44. This specificity preferably promotes the recruitment of T cells.

Preferably, the T cell surface antigen is CD3. It means that the antibody for use according to the present invention further preferably comprises a paratope which can recognize and bind to an epitope of CD3.

Catumaxomab, an example for a bispecific antibody, binds EpCAM-positive tumor cells and CD3-positive T-cells through its 2 specific binding sites. Catumaxomab also recruits FcyR type I, IIa and III-positive accessory cells via binding of its intact fragment crystallizable (Fc) region resulting in a trifunctional mode of action. Beyond the mode of action of Catumaxomab when used in the present invention, several mechanisms of tumor cell destruction induced by bispecific trifunctional antibodies have been described.

Zeidler et al. [0], [0], [0] and Riechelmann et al. [0] as listed blow have elucidated the activation of T-cells and accessory cells in vitro, and their contribution to tumor cell killing, by BiUII (which is a variant antibody to Catumaxomab) and Catumaxomab. Using this trifunctional antibody with peripheral blood mononuclear cells (PBMCs), they showed that accessory cell activation is an important contributor to the antitumor activity. Activation of T-cells and accessory cells led to production of cytokines (interleukin [IL]-1β, IL-2, IL-6, IL-12, TNF-α, interferon-γ [IFN-y] and Chemokine (C-C motif) ligand 18 [CCL18]). Remarkably, Riechelmann et al. demonstrated that IFN-y reached peak values within 5 hours and TNF-α within 24 hours. They also demonstrated the upregulation of activation markers on dendritic cells and NK cells. Catumaxomab did not bind to B-lymphocytes, but it stimulated FcyR-positive accessory cells to eliminate tumor cells by direct phagocytosis.

[53] Zeidler R, Mayer A, Gires O, et al. TNF-alpha contributes to the anti-tumor activity of a bispecific, trifunctional antibody. Anticancer Res. 2001;21(5):3499-3503.

[54] Zeidler R, Mysliwietz J, Csanady M, et al. The Fc-region of a new class of intact bispecific antibody mediates activation of accessory cells and NK cells and induces direct phagocytosis of tumor cells. Br J Cancer. 2000;83(2):261-266.

[55] Zeidler R, Reisbach G, Wollenberg B, et al. Simultaneous activation of T-cells and accessory cells by a new class of intact bi- specific antibody results in efficient tumor cell killing. J Immunol 1999;163(3):1246-1252.

[36] Riechelmann H, Wiesneth M, Schauwecker P, et al. Adoptive therapy of head and neck squamous cell carcinoma with antibody coated immune cells: a pilot clinical trial. Cancer Immunol Immunother. 2007;56(9):1397-1406.

[1] Abbas AK, Lichtman AH. General properties of cytokines. In: Cellular and Molecular Immunology. 5th edition. Philadephia, Pennsylvania: Saunders, imprint of Elsevier; 2003: Section IV.

In a preferred embodiment, the antibodies used in the present invention are monoclonal antibodies. This is specifically true for the bispecific antibodies disclosed herein in detail.

Proteins having relatively defined three-dimensional structures are commonly referred to as protein scaffolds. These protein scaffolds may be used as reagents for the design of artificially engineered antibodies. These scaffolds typically contain one or more regions which are amenable to specific or random sequence variation, and such sequence randomization is often carried out to produce libraries of proteins from which the desired antibody scaffolds may be selected. Such scaffolds are particularly useful in the field of antibody design.

These antibody scaffolds are non-immunoglobulin proteins which mimic properties of a monoclonal antibody with respect to its binding activity to for instance tumor cells and immune cells. Scaffolds often include loops or domains which form the binding side of said antibody scaffold. These antibody mimics may be utilized for the purpose of designing proteins which are capable of binding to virtually any compound of interest. This directed evolution approach results in the production of antibody-like molecules with high affinities for antigens of interest. In addition, those scaffolds may be used to display defined exposed loops (e.g. loops previously randomized and selected on the basis of antigen binding) in order to direct evolution of molecules that bind to such introduced loops. Methods on how to obtain antibody-like scaffold proteins are known in the art. The following describes one possible approach for obtaining an antibody-like scaffold protein.

A first screening method, useful for the isolation or identification of randomized or mutated proteins of interest, involves: (a) contacting a compound of interest with a candidate protein, the candidate protein being a derivative non-antibody protein including a domain having an immunoglobulin-like fold, the non-antibody protein deriving from a reference protein by having a mutated amino acid sequence wherein the non-antibody protein binds with a Kd at least as tight as 1 microM to a compound that is not bound as tightly by the reference protein, wherein the contacting is carried out under conditions that allow compound-protein complex formation; and (b) obtaining, from the complex, the derivative protein that binds to the compound.

The second screening method is for isolating or identifying a compound which binds to a tumor-associated protein of interest. This method begins with a non-antibody protein including a domain having an immunoglobulin-like fold and deriving from a reference protein by having a mutated amino acid sequence, wherein the non-antibody protein binds with a Kd at least as tight as 1 µM to a compound that is not bound as tightly by the reference protein. This derivative protein is then contacted with a candidate compound (tumor-associated antigen or an epitope thereof), wherein the contacting is carried out under conditions that allow compound-protein complex formation, and the compound which binds to the derivative protein is obtained from the complex. Again, this general technique may be carried out with any protein.

Further methods of obtaining non-antibody proteins which bind to compounds of interest (tumor-associated antigen or an epitope thereof) are described as follows. One such method involves: (a) providing a non-antibody scaffold protein including an immunoglobulin-like fold, wherein the scaffold protein does not bind to the compound with a Kd as tight as 1 micro M; (b) generating mutated derivatives of the non-antibody scaffold protein, thereby producing a library of mutated proteins; (c) contacting the library with the compound; (d) selecting from the library at least one derivative protein which binds to the compound with a Kd at least as tight as 1 µM; and (e) optionally repeating steps (b)- (d) substituting for the non-antibody scaffold protein in repeated step (b) the product from the previous step (d). Again, this general technique may be carried out with any protein.

The so produced scaffold proteins mimic the function of an antibody as disclosed above and below and can be used either instead of an immunoglobulin-based antibody or in combination with it.

Preferably, the Fc-portion of the present invention displays substantially low or no complement binding to complement factors, which may be connected with a better effect in respect of no or only marginal complement dependent cytotoxicity at antibody concentrations <1000ng/ml, more preferably at antibody concentrations <500ng/ml, and with an improved avoidance of nerve pain.

Preferably, above complement factors may be Fc receptor or complement protein component 1 q (C1q).

Generally, an antibody may comprise an Fc-portion which can bind to the Fc receptor-positive cell via Fcγ receptors of type I, II and III, and said Fc-portion may comprise at least one binding site for said Fcγ receptor type I, II and/or III.

Preferably, the antibody used in the present invention comprises an Fc-portion wherein the complement binding amino acids in said Fc-protein are modified by one of substitutions, insertions or deletions of amino acids or a combination thereof. In this regard, a better effect in respect of no or only marginal complement dependent cytotoxicity at antibody concentrations <100ng/ml, more preferably at antibody concentrations <500ng/ml, can be achieved and an improved avoidance of nerve pain can be achieved.

Preferably, the complement binding amino acids of above Fc-portion comprise an ELLGGP motif (SEQ ID NO: 1), more preferably an E233 L234 L235 G236 G237 P238 motif, an EFLGGP motif (SEQ ID NO: 2), more preferably an E233 F234 L235 G236 G237 P238 motif, or an PVAGGP motif (SEQ ID NO: 3), more preferably an P233 V234 A235 G236 G237 P238 motif. Said motif sequences E233 L234 L235 G236 G237 P238, E233 F234 L235 G236 G237 P238 and P233 V234 A235 G236 G237 P238 are disclosed by John D. Issacs et al. (J Immunol, 1998, 161, pages 3862-3869), and the essential information with regard to said sequences is referred to said publication of John D. Issacs et al..

Preferably, the ELLGGP motif of above Fc-portion, more preferably the E233 L234 L235 G236 G237 P238 motif, is modified by one of substitutions, insertions or deletions of amino acids or a combination thereof, wherein the antibody comprising said Fc portion is preferably a human IgG1 or IgG3 antibody. In this regard, a better effect in respect of no or only marginal complement dependent cytotoxicity at antibody concentrations <100ng/ml, more preferably at antibody concentrations <500ng/ml, can be achieved and an improved avoidance of nerve pain can be achieved.

Preferably, the EFLGGP motif of above Fc-portion, more preferably the E233 F234 L235 G236 G237 P238 motif of above Fc-portion, is modified by one of substitutions, insertions or deletions of amino acids or a combination thereof, wherein the antibody comprising said Fc-portion is preferably a human IgG4 antibody. In this regard, a better effect in respect of no or only marginal complement dependent cytotoxicity at antibody concentrations <100ng/ml, more preferably at antibody concentrations <500ng/ml, can be achieved and an improved avoidance of nerve pain can be achieved.

Preferably, the PVAGGP motif of above Fc-portion, more preferably the P233 V234 A235 G236 G237 P238 motif of above Fc-portion, is modified by one of substitutions, insertions or deletions of amino acids or a combination thereof, wherein the antibody comprising said Fc-portion is preferably a human IgG2 antibody. In this regard, a better effect in respect of no or only marginal complement dependent cytotoxicity at antibody concentrations <100ng/ml, more preferably at antibody concentrations <500ng/ml, can be achieved and an improved avoidance of nerve pain can be achieved.

Preferably, the antibody comprising above modified Fc-portion is a mouse IgG1-IgG4 or a human IgG1-IgG4 antibody. In this regard, a better effect in respect of no or only marginal complement dependent cytotoxicity at antibody concentrations <100ng/ml, more preferably at antibody concentrations <500ng/ml, can be achieved and an improved avoidance of nerve pain can be achieved.

Preferably, the ELL or EFL of above SEQ ID NO: 1 or SEQ ID NO: 2 motifs of Fc-Portion, more preferably the E233 L234 L235 or E233 F234 L235 of above motifs, is modified by substitution, wherein the substituted motif is preferably PVA (SEQ ID NO: 4), more preferably the substituted motif is E233P L234V L235A or E233P F234V L235A. In this regard, a better effect in respect of no or only marginal complement dependent cytotoxicity at antibody concentrations <100ng/ml, more preferably at antibody concentrations <500ng/ml, can be achieved and an improved avoidance of nerve pain can be achieved.

Preferably, the antibody used in the present invention comprises a Fc-portion which displays substantially low or no complement binding to C1q, which may be connected with a better effect in respect of no or only marginal complement dependent cytotoxicity at antibody concentrations <100ng/ml, more preferably at antibody concentrations <500ng/ml, and with an improved avoidance of nerve pain.

Preferably, the antibody used in the present invention may comprise a substitution or deletion at position P329, or an insertion adjacent to said P329, which may be connected with a better effect in respect of no or only marginal complement dependent cytotoxicity at antibody concentrations <100ng/ml, more preferably at antibody concentrations <500ng/ml, and with an improved avoidance of nerve pain. Said position P329 is disclosed by Tilman Schlothauer et al. (Protein Engineering, Design & Selection, 2016, vol. 29, no. 10, pages 457-466), and the essential information with regard to said P329 is referred to said publication of Tilman Schlothauer et al..

Preferably, the antibody used in the present invention may comprise a substitution or deletion at position P329, or an insertion adjacent to said P329, which may render the Fc-portion of the antibody displaying substantially low or no complement binding to C1q.

Preferably, the antibody used in the present invention may comprise a substitution P329A or P329G, more preferably P329G, which may be connected with a better effect in respect of no or only marginal complement dependent cytotoxicity at antibody concentrations <100ng/ml, more preferably at antibody concentrations <500ng/ml, and with an improved avoidance of nerve pain.

Preferably, the antibody used in the present invention may comprise a substitution P329A or P329G, more preferably P329G, which may render the Fc-portion of the antibody displaying substantially low or no complement binding to C1q.

Preferably, said substitution or deletion at position P329, or said insertion adjacent to said P329, is present in combination with above ELLGGP, EFLGGP, or PVAGGP motif, more preferably is present in combination with above E233 L234 L235 G236 G237 P238 motif, E233 F234 L235 G236 G237 P238 motif, or P233 V234 A235 G236 G237 P238 motif.

Preferably, said substitution or deletion at position P329, or said insertion adjacent to said P329, is present in combination with above E233 L234 L235 or E233 F234 L235 substitution.

Preferably, said substitution or deletion at position P329, or said insertion adjacent to said P329, is present in combination with L234A L235A substitution.

Preferably, above substitution P329A or P329G, more preferably P329G, is present in combination with ELLGGP, EFLGGP, or PVAGGP motif, more preferably is present in combination with above E233 L234 L235 G236 G237 P238 motif, E233 F234 L235 G236 G237 P238 motif, or P233 V234 A235 G236 G237 P238 motif.

Preferably, above substitution P329A or P329G, more preferably P329G, is present in combination with above E233 L234 L235 or E233 F234 L235 substitution.

Preferably, above substitution P329A or P329G, more preferably P329G, is present in combination with L234A L235A substitution.

Preferably, the antibody used according to the invention is able to bind to monocytes, macrophages, dendritic cells, natural killer cells, neutrophils and/or eosinophilic cells being Fcγ receptor positive cells.

In the present invention, the tumor associated antigen refers to an antigenic substance produced in tumor cells which is expressed on the surface of a tumor cell.

Preferably, the tumor associated antigen that the bispecific antibody binds to is selected from the group consisting of tumor associated sphingolipids and sphingoid molecules, gangliosides or glucosphingolipids like e.g. GD2, GD3, GM1, GM2, GM3, fucosyl-GM1, globo-H, S1P, Cer, and Gg3, preferably the tumor-associated antigen is the neuroblastoma-associated antigen GD2.

Preferably, the tumor associated antigen that the bispecific antibody binds to is selected from the group consisting of tumor associated sphingolipids and sphingoid molecules, gangliosides or glucosphingolipids such as GD2, GD3, GM1, GM2, GM3, fucosyl-GM1, globo-H, S1P, Cer, and Gg3, preferably the tumor-associated antigen is the neuroblastoma-associated antigen GD2.

Preferably, the bispecific antibody binds to tumor-associated antigens sphingolipids and sphingoid molecules like e.g. gangliosides, which causes no or reduced nerve pain in the treatment of cancer.

Preferably, the bispecific antibody binds to tumor-associated antigens sphingolipids and sphingoid molecules such as gangliosides, which causes no or reduced nerve pain in the treatment of cancer.

Preferably, the tumor-associated antigen is the neuroblastoma-associated antigen GD2, which is connected with a better effect in respect of no or only marginal complement dependent cytotoxicity at antibody concentrations <100ng/ml, more preferably at antibody concentrations <500ng/ml, and with an improved avoidance of nerve pain. This may be attributed to the monovalent binding of the presently disclosed antibody with the GD2 binding site.

Preferably, the bispecific antibody of the present invention is directed against one T cell surface antigen and one tumor-associated antigen, wherein the T cell surface antigen which is selected from a group consisting of CD2, CD3, CD4, CD8, CD28, CD40L and CD44, can be combined with any tumor-associated antigen selected from a group consisting of tumor associated sphingolipids and sphingoid molecules, gangliosides or glucosphingolipids like e.g. GD2, GD3, GM1, GM2, GM3, fucosyl-GM1, globo-H, S1P, Cer, and Gg3, preferably the tumor-associated antigen is the neuroblastoma-associated antigen GD2.

Preferably, the bispecific antibody of the present invention is directed against one T cell surface antigen and one tumor-associated antigen, wherein the T cell surface antigen which is selected from a group consisting of CD2, CD3, CD4, CD8, CD28, CD40L and CD44, can be combined with any tumor-associated antigen selected from a group consisting of tumor associated sphingolipids and sphingoid molecules, gangliosides or glucosphingolipids such as GD2, GD3, GM1, GM2, GM3, fucosyl-GM1, globo-H, S1P, Cer, and Gg3, preferably the tumor-associated antigen is the neuroblastoma-associated antigen GD2.

Preferably, the bispecific antibody of the present invention is directed against one T cell surface antigen which is CD3 and one tumor-associated antigen which is GD2.

Preferred antibodies are heterologous bispecific antibodies, preferably monoclonal, selected from one or more of the following combinations of isotypes:
- rat-IgG2b/mouse-IgG2a,
- rat-IgG2b/mouse-IgG2b,
- rat-IgG2b/mouse-IgG3;
- rat-IgG2b/human-IgG1,
- rat-IgG2b/human-IgG2
- rat-IgG2b/human-IgG3 [oriental allotype G3m(st)=binding to protein A], rat-IgG2b/human-IgG4;
- rat-IgG2b/rat-IgG2c;
- mouse-IgG2a/human-IgG3 [caucasian allotypes G3m(b+g)=no binding to protein A, in the following indicated as *]
- mouse-IgG2a/mouse-[VH-CH1, VL-CL]-human-IgG1-[hinge]-human-IgG3*-[CH2-CH3]
- mouse-IgG2a/rat-[VH-CH1, VL-CL]-human-IgG1-[hinge]-human-IgG3*-[CH2-CH3]
- mouse-IgG2a/human-[VH-CH1, VL-CL]-human-IgG1-[hinge]-human-IgG3*-[CH2-CH3]
- mouse-[VH-CH1, VL-CL]-human-IgG1/rat-[VH-CH1, VL-CL]-humanIgG1-[hinge]-human-IgG3*-[CH2-CH3]
- mouse-[VH-CH1, VL-CL]-human-IgG4/rat-[VH-CH1, VL-CL]-human-IgG4-[hinge]-human-IgG4 [N-terminal region of CH2]-human-IgG3*[C-terminal region of CH2:>aa position 251]-human-IgG3*[CH3]
- at-IgG2b/mouse-[VH-CH1, VL-CL]-human-IgG1-[hinge-CH2-CH3]
- rat-IgG2b/mouse-[VH-CH1, VL-CL]-human-IgG2-[hinge-CH2-CH3]
- rat-IgG2b/mouse-[VH-CH1, VL-CL]-human-IgG3-[hinge-CH2-CH3, oriental allotype]
- rat-IgG2b/mouse-[VH-CH1, VL-CL]-human-IgG4-[hinge-CH2-CH3]
- human-IgG1/human-[VH-CH1, VL-CL]-human-IgG1-[hinge]-human-IgG3*-[CH2-CH3]
- human-IgG1/rat-[VH-CH1, VL-CL]-human-IgG1-[hinge]-human-IgG4 [N-terminal region of CH2]-human-IgG3*[C-terminal region of CH2:>aa position 251]-human-IgG3*[CH3]
- human-IgG1/mouse-[VH-CH1, VL-CL]-human-IgG1-[hinge]-human-IgG4 [N-terminal region of CH2]-human-IgG3*[C-terminal region of CH2:>aa position 251]-human-IgG3*[CH3]
- human-IgG1/rat-[VH-CH1, VL-CL]-human-IgG1-[hinge]-human-IgG2 [N-terminal region of CH2]-human-IgG3*[C-terminal region of CH2:>aa position 251]-human-IgG3*[CH3] human-IgG1/mouse-[VH-CH1, VL-CL]-human-IgG1-[hinge]-human-IgG2 [N-terminal region of CH2]-human-IgG3*[C-terminal region of CH2:>aa position 251]-human-IgG3*[CH3]
- human-IgG1/rat-[VH-CH1, VL-CL]-human-IgG1-[hinge]-human-IgG3*-[CH2-CH3]
- human-IgG1/mouse-[VH-CH1, VL-CL]-human-IgG1-[hinge]-human-IgG3*-[CH2-CH3]
- human-IgG2/human-[VH-CH1, VL-CL]-human-IgG2-[hinge]-human-IgG3*-[CH2-CH3]
- human-IgG4/human-[VH-CH1, VL-CL]-human-IgG4-[hinge]-human-IgG3*-[CH2-CH3]
- human-IgG4/human-[VH-CH1, VL-CL]-human-IgG4-[hinge]-human-IgG4 [N-terminal region of CH2]-human-IgG3*[C-terminal region of CH2:>aa position 251]-human-IgG3*[CH3]
- mouse-IgG2b/rat-[VH-CH1, VL-CL]-human-IgG1-[hinge]-human-IgG3*-[CH2-CH3]
- mouse-IgG2b/human-[VH-CH1, VL-CL]-human-IgG1-[hinge]-human-IgG3*-[CH2-CH3]
- mouse-IgG2b/mouse-[VH-CH1, VL-CL]-human-IgG1-[hinge]-human-IgG3*-[CH2-CH3]

The bispecific antibody preferably with monovalent or bivalent binding specificities used in one particularly preferred embodiment by the present invention has the following properties:
An intact whole IgG bispecific antibody, comprising the following properties:
   a) binding to a T cell;
   b) binding to a tumor-associated antigen on a tumor cell;
   c) binding via its Fc-portion to a Fc-receptor positive cell,
   d) no or only marginal complement dependent cytotoxicity at antibody concentrations <500ng/ml resulting in the avoidance of nerve pain, wherein the bispecific antibody is further selected from a group of antibodies with the following isotype combinations:
rat-IgG2b/mouse-IgG2a,
rat-IgG2b/mouse-IgG2b,
rat-IgG2b/human-IgG1,
mouse-[VH-CH1; VL-CL]-human-IgG1/rat-[VH-CH1, VL-CL]-human-IgG1-[hinge]-human-IgG3*-[CH2-CH3]
[* = Caucasian allotypes G3m(b+g) = no binding to protein A]. This preferred antibody can be connected with a better effect in respect of no or only marginal complement dependent cytotoxicity at antibody concentrations <500ng/ml and with an improved avoidance of nerve pain. This may be attributed to the monovalent binding of the presently disclosed antibody with the GD2 binding site.

Specifically preferred is an antibody, preferably a bispecific antibody and/or a scaffold protein directed against GD2 and CD3 with the isotype combination rat-IgG2b/mouse-IgG2a. A preferred example for said bispecific antibody is anti-CD3 x anti-GD2 antibody. Preferably said antibody is monoclonal.

Preferably, the antibodies according to the invention are monoclonal, chimeric, recombinant, synthetic, semi-synthetic, or chemically modified intact antibodies having for example Fv, Fab, scFv, or F (ab)2 fragments.

In the method of the present invention also antibodies or derivatives or fragments of human origin can be used, or antibodies modified to be suitable for the use in humans (so-called "humanized antibodies") (see for example Shalaby et al., J. Exp. Med. 175 (1992), 217; Mocikat et al., Transplantation 57 (1994), 405).

The preparation of the different types of antibodies and antibody fragments mentioned above is obvious to the skilled artisan. The preparation of monoclonal antibodies preferably of mammalian origin, e.g. of human, rat, mouse, rabbit, or goat, can be performed using conventional methods for example as those described in Köhler and Milstein (Nature 256 (1975), 495), in Harlow and Lane (Antibodies, A Laboratory Manual (1988), Cold Spring Harbor) or in Galfré (Meth. Enzymol. 73 (1981), 3).

It is further possible to prepare the antibodies described by means of recombinant DNA technology according to techniques obvious to the skilled artisan (see Kurucz et al., J. Immunol. 154 (1995), 4576; Hollinger et al., Proc. Natl. Acad. Sci. USA 90 (1993), 6444).

The preparation of antibodies having two different specificities, the so-called bispecific antibodies, can be performed for example using recombinant DNA technology but also by the so-called hybrid hybridoma fusion technique (see for example Milstein et al., Nature 305 (1983), 537). This technique comprises fusing hybridoma cell lines each producing antibodies having one of the desired specificities and identifying and isolating recombinant cell lines producing antibodies having both specificities.

The problem forming the basis of the invention can be overcome by using in preferred embodiments either bispecific or trispecific antibodies if they exhibit the properties and effects as described herein. The invention is particularly described by the way of bispecific antibodies. However, it is understood that it also covers the following trispecific antibodies exhibiting similar effects. Although above, the terms "antibody" or "scaffold protein" may refer to bispecific antibody, it is understood that it can also covers the following trispecific antibodies exhibiting similar effects.

The preparation of antibodies exhibiting three specificities, so-called trispecific antibodies, also suitable to solve the problem of the invention, may for example be carried out by coupling a third antigen binding site having an additional specificity, e.g. in the form of "single chain variable fragments" (scFv) to one of the IgG heavy chains of a bispecific antibody. Further, recombinant technology can be used, e.g. vector-based methods for protein synthesis or oligonucleotide synthesis.

Analogously, trispecific F(ab)2 constructs may be prepared by replacing the CH2-CH3 regions of the heavy chain of one specificity of a bispecific antibody by an scFv having a third specificity, while the CH2-CH3 regions of the heavy chain having the other specificity can be removed for example by insertion of a stop codon (at the end of the "hinge" 5 region) into the coding gene, e.g by homologous recombination.

It is also possible to prepare trispecific scFv constructs wherein three VH-VL regions representing three different specificities are arranged in series.

Intact bispecific antibodies are composed of two antibody semi-molecules (each having a H and a L immunoglobulin chain) each representing a specificity, and additionally like normal antibodies having a Fc portion performing the well-known effector functions. They are preferably prepared using the quadroma technology. This method of preparation is exemplified in DE-A-44 19 399. For complete disclosure this document is incorporated in its entirety by reference also with respect to a definition of bispecific antibodies. It should be understood that other methods of preparation are also useful if they lead to the intact bispecific antibodies according to the above definition required according to the invention.

For example, intact bispecific antibodies may be produced in sufficient amounts using a newly developed method of preparation (Lindhofer et al., J. Immunology, 155:219 (1995)). The combination of two bispecific antibodies directed against two different tumor-associated antigens (e.g. c-erb-B2, EpCAM, such as GA-733-2=C215) on the mammary carcinoma cells minimizes the risk that tumor cells expressing only one of the antigens remain unidentified.

### Figures

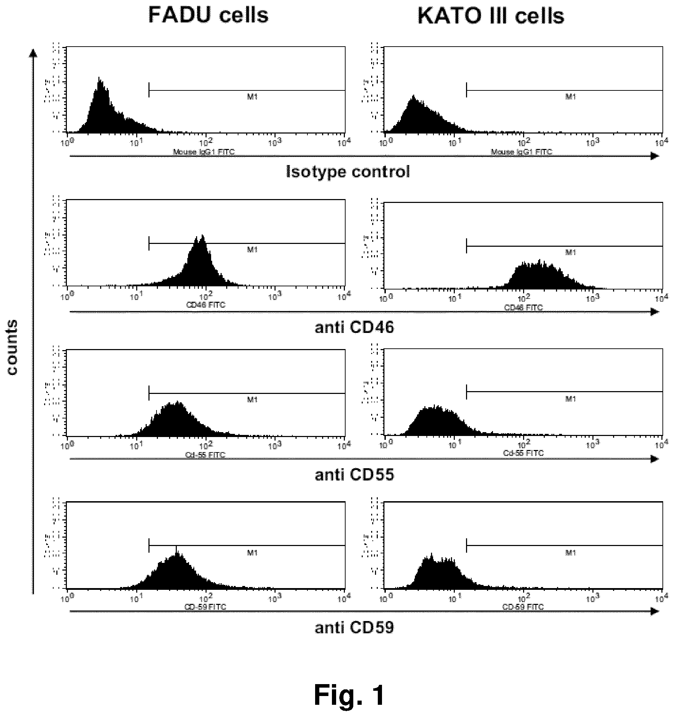

Figure 1. Flow cytometric analysis of mCRP expression by FADU and KATO III cells. FADU cells and KATO III cells were stained with FITC-conjugated antibodies directed against mCRPs (CD46, CD55, CD59) or with an isotype control. Histograms show FITC-fluorescence intensity as measured by flow cytometry.

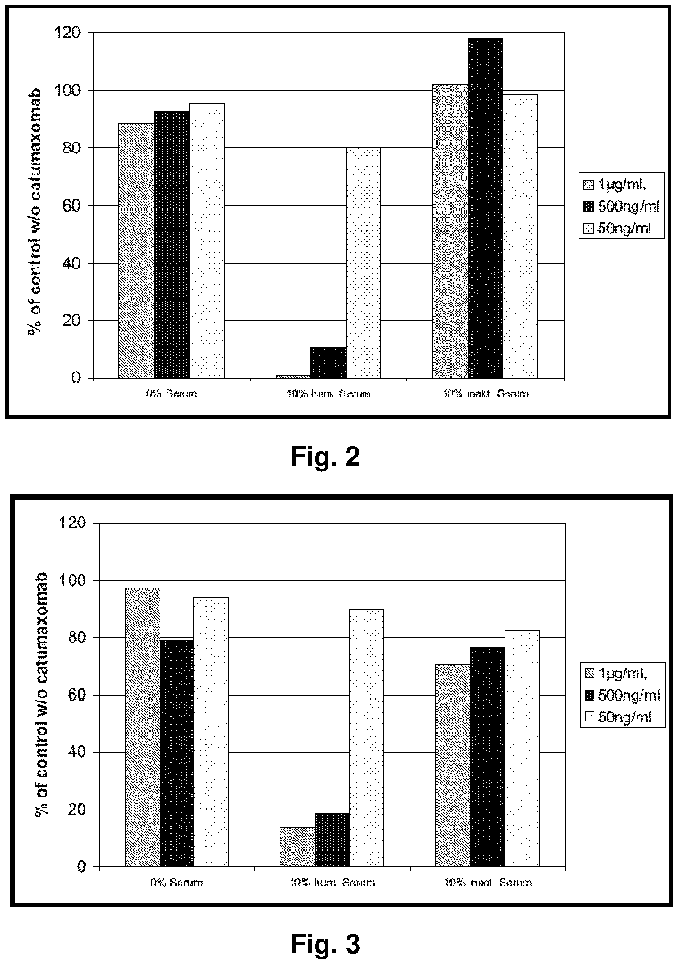

Figure 2. Analysis of complement dependent cytotoxicity mediated by catumaxomab from 31.08.05, donor I. KATO III cells were incubated with 50 ng/ml, 500 ng/ml or 1 µg/ml catumaxomab and either 10% normal serum (hum. serum), 10% inactivated serum (inact. serum) or without serum. Tumor cell survival is indicated as percent of controls without catumaxomab. w/o = without
Figure 3. Analysis of complement dependent cytotoxicity mediated by catumaxomab from 31.08.05, donor II. KATO III cells were incubated with 50 ng/ml, 500 ng/ml or 1 µg/ml catumaxomab and either 10% normal serum (hum. serum), 10% inactivated serum (inact. serum) or without serum. Tumor cell survival is indicated as percent of controls without catumaxomab. w/o = without

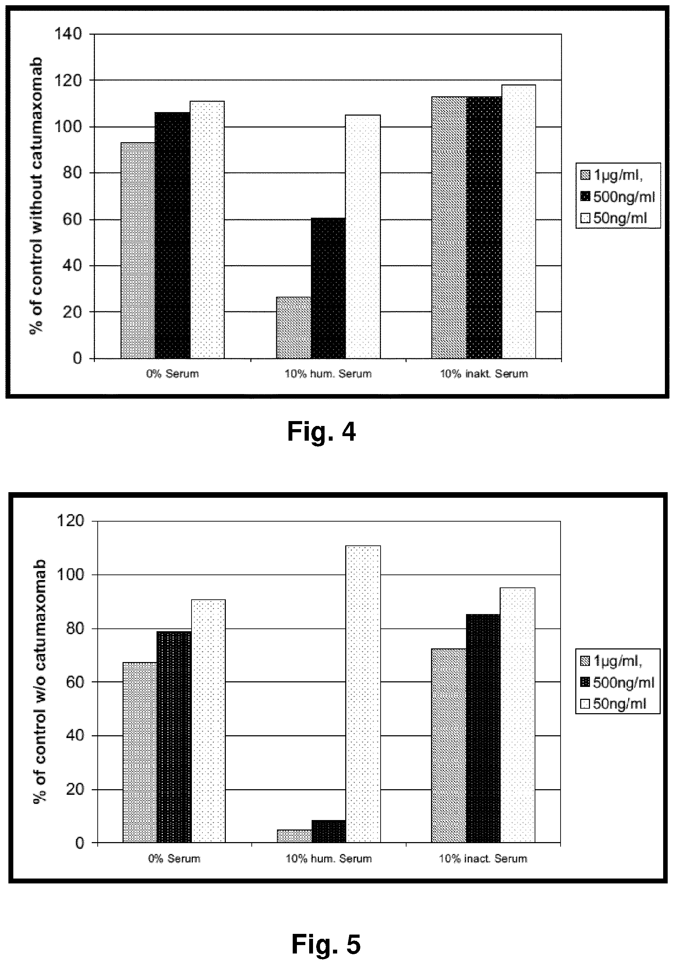

Figure 4. Analysis of complement dependent cytotoxicity mediated by catumaxomab from 24.08.05, donor III. KATO III cells were incubated with 50 ng/ml, 500 ng/ml or 1 µg/ml catumaxomab and either 10% normal serum (hum. serum), 10% inactivated serum (inact. serum) or without serum. Tumor cell survival is indicated as percent of controls without catumaxomab. w/o = without
Figure 5. Analysis of complement dependent cytotoxicity mediated by catumaxomab from 20.07.05, donor IV. KATO III cells were incubated with 50 ng/ml, 500 ng/ml or 1 µg/ml catumaxomab and either 10% normal serum (hum. serum), 10% inactivated serum (inact. serum) or without serum. Tumor cell survival is indicated as percent of controls without catumaxomab. w/o = without

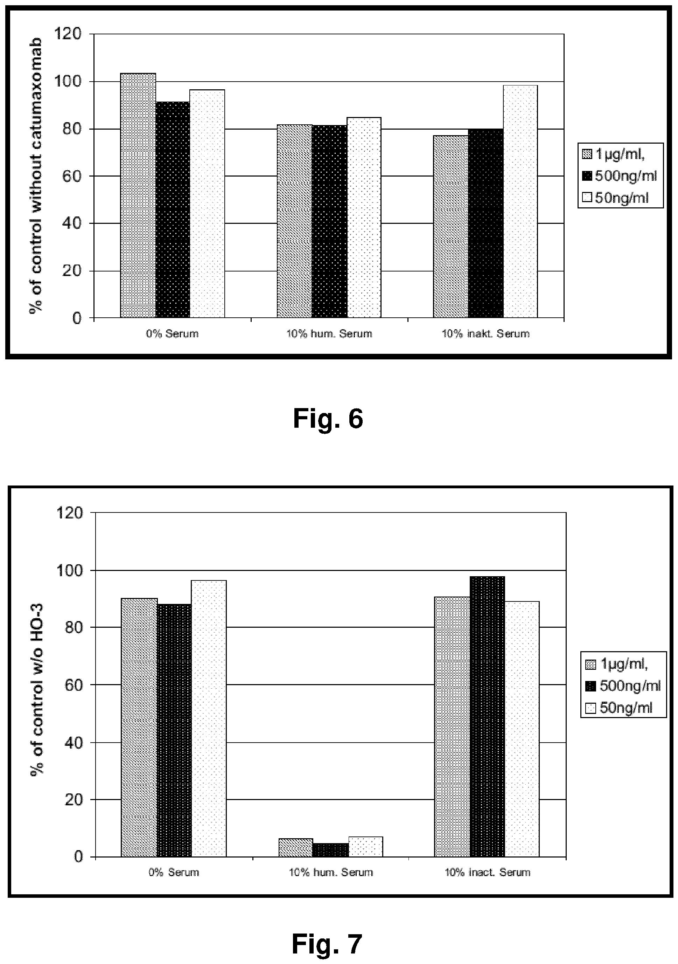

Figure 6. Analysis of complement dependent cytotoxicity mediated by catumaxomab from 20.07.05, donor IV. FADU cells were incubated with 50 ng/ml, 500 ng/ml or 1 µg/ml catumaxomab and either 10% normal serum (hum. serum), 10% inactivated serum (inact. serum) or without serum. Tumor cell survival is indicated as percent of controls without catumaxomab. w/o = without
Figure 7. Analysis of complement dependent cytotoxicity mediated by HO-3 from 31.08.05, donor I. KATO III cells were incubated with 50 ng/ml, 500 ng/ml or 1 µg/mlcatumaxomab and either 10% normal serum (hum. serum), 10% inactivated serum (inact. serum) or without serum. Tumor cell survival is indicated as percent of controls without HO-3. w/o = without

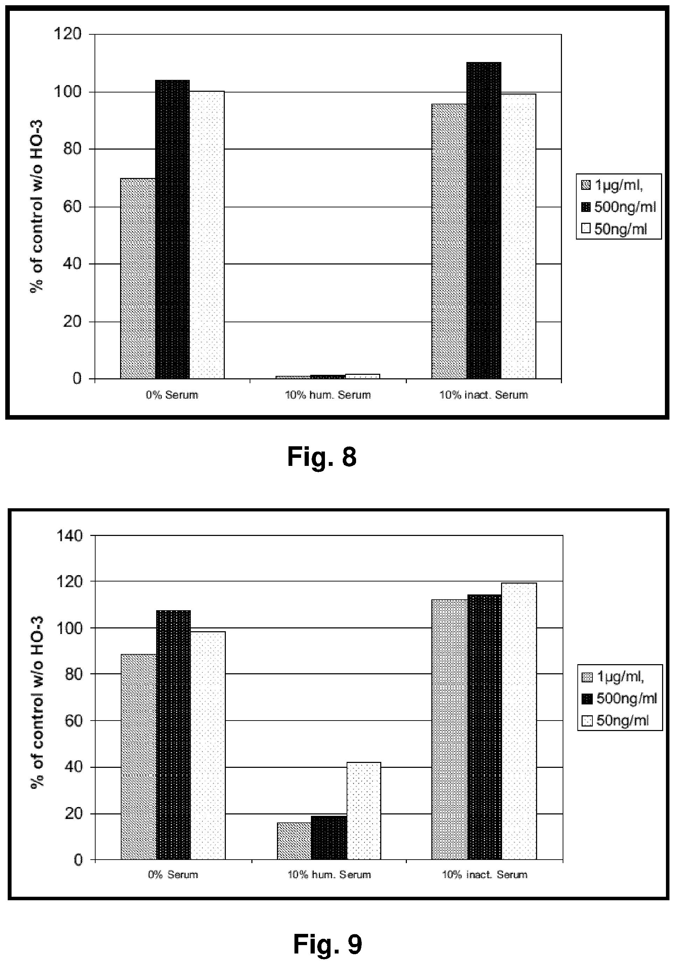

Figure 8. Analysis of complement dependent cytotoxicity mediated by HO-3 from 31.08.05, donor II. KATO III cells were incubated with 50 ng/ml, 500 ng/ml or 1 µg/mlcatumaxomab and either 10% normal serum (hum. serum), 10% inactivated serum (inact. serum) or without serum. Tumor cell survival is indicated as percent of controls without HO-3. w/o = without
Figure 9. Analysis of complement dependent cytotoxicity mediated by HO-3 from 24.08.05, donor III. KATO III cells were incubated with 50 ng/ml, 500 ng/ml or 1 µg/mlcatumaxomab and either 10% normal serum (hum. serum), 10% inactivated serum (inact. serum) or without serum. Tumor cell survival is indicated as percent of controls without HO-3. w/o = without

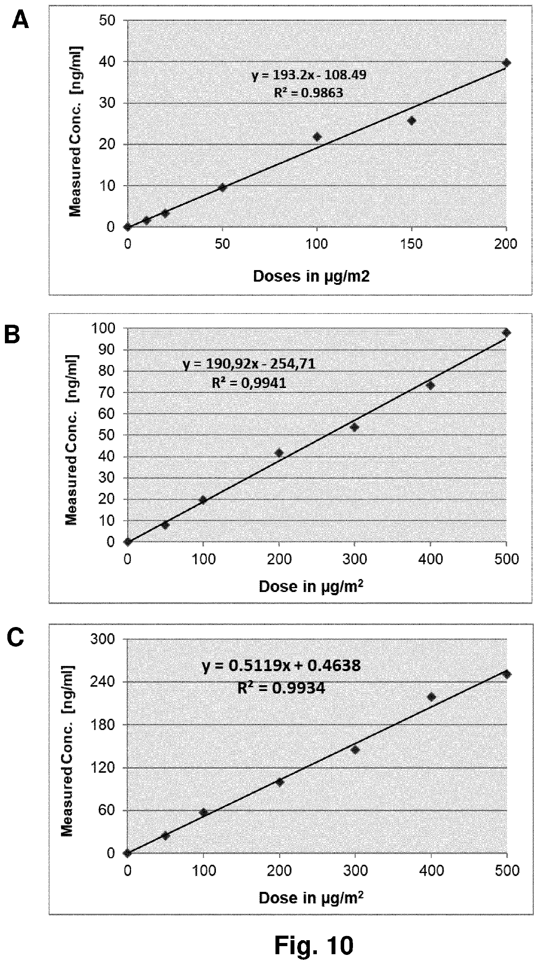

Figure 10. A Grade IV neuroblastoma Patient 1 with bone metastasis was treated with two cycles of increasing amounts of the bispecific antibody EKTOMUN. Pharmacokinetic analysis reveals dose linearity with increasing amount of circulating antibody for Patient 1 as well as for another Patient 2. (A) First treatment cycle for Patient 1; (B) Second treatment cycle for Patient 1; (C) First treatment cycle for Patient 2.

In the following, one exemplary example for carrying out the invention is described. A person with ordinary skill in the art following this approach will inevitably result in obtaining the claimed benefit. Following this example, various modifications can be performed without deviating from the invention. The following Example is merely for illustrating the above disclosure, and should not be seen as limiting the scope of present invention.

### Examples

### Example 1

To compare the complement binding activities of the parental anti-EpCAM antibody HO-3 (isotype mouse IgG2a) with the bispecific antibody Catumaxomab (anti-EpCAM x anti-CD3) with the isotype combination mouse IgG2a X rat IgG2b, experiments were performed to assess complement-mediated cytotoxicity as a model system for Ektomun and the related parental antibody Me361 which possess the identical Fc regions and therefore complement binding characteristics. Moreover, the CD3-binding arms of Catumaxomab and of Ektomun are identical.

Firstly, as a prerequisite, the expression of the membrane-bound complement regulating proteins (mCRP) on the surface of the tumor target cell lines KATO III and FADU was measured in flow cytometry (Figure 1).

Membrane-bound complement regulatory proteins (mCRPs), such as CD46, CD55, and CD59, are expressed throughout the body in order to prevent over-activation of the complement system. These mCRPs act as a double-edged sword however, as they can also over-regulate the complement system to the extent that it is no longer effective at eliminating cancerous cells (Geller A and Yan J (2079) The Role of Membrane Bound Complement Regulatory Proteins in Tumor Development and Cancer Immunotherapy. Front. Immunol. 10:1074. doi: 10.3389/fimmu.2019.01074 ).

Therefore, to better understand the complement dependent cytotoxicity of tested antibodies, which was measured in a further set of experiments, the expression of mCRPS was assessed.

Secondly, the complement dependent cytotoxicity of the antibody Catumaxomab was analysed with KATOIII with 4 different complement donors and with FADU using one complement donor (Figures 2-6).

Complement dependent cytotoxicity mediated by the parental anti-EpCAM antibody HO-3 was also assessed based on the similar methods as explained above (Figures 7-9).

### Example 2

Treatment of Neuroblastoma patients on a named patient basis with the bispecific antibody EKTOMUN (anti-GD2 x anti-CD3) did not cause nerve pain. A Grade IV neuroblastoma Patient 1 with bone metastasis was treated with two cycles of increasing amounts of the bsAb EKTOMUN (Figure 10A-B). Pharmacokinetic analysis reveals dose linearity with increasing amount of circulating antibody for Patient 1 as well as for another Patient 2 (Figure 10A-C). For Patient 1 the first treatment cycle all adverse events were closely documented (see Tables 1-3 below). Expected antibody-related adverse events were fever, chills and flu-like symptoms. However, no antibody-related nerve pain was observed. From personal communication it is known that also during and after the second treatment cycle no nerve pain occurred.

### Toxicity Evaluation of Neuroblastoma patient treated with EKTOMUN (i.v.)

**Table 1**

| **Treatment Day** | **Adverse event** | **Grade** | **Related to EKTOMUN** |
|---|---|---|---|
| 21.05.2012 | | | |
| **Dosis [µg/m2]** | White Blood cell decresed | 1 | unlikely |
| 10 | Neurophil count decreased | 0 | |
| | Lymphocyte count decreased | 3 | unlikely |
| | Anemia | 3 | unlikely |
| | Platelet count decreased | 3 | unlikely |
| | ALAT | 1 | possible |
| | Fatigue | 1 | unlikely |
| | Hypotension | 1 | unlikely |
| | Abdominal Pain | 0 | |
| | Fever | 0 | |

| | | | |
|---|---|---|---|
| | | | |

| **Treatment Day** | **Adverse event** | **Grade** | **Related to EKTOMUN** |
|---|---|---|---|
| 24.05.2012 | | | |
| **Dosis [µg/m2]** | White Blood cell decresed | 1 | unlikely |
| 20 | Neurophil count decreased | 0 | |
| | Lymphocyte count decreased | 3 | unlikely |
| | Anemia | 1 | unlikely |
| | Platelet count decreased | 3 | unlikely |
| | ALAT | 0 | |
| | Fatique | 1 | unlikely |
| | Hypotension | 1 | unlikely |
| | Abdominal Pain | 1 | unlikely |
| | Fever | 0 | |

| **Treatment Day** | **Adverse event** | **Grade** | **Related to EKTOMUN** |
|---|---|---|---|
| 28.05.2012 | | | |
| **Dosis [µq/m2]** | White Blood cell decresed | 0 | unlikely |
| 50 | Neurophil count decreased | 0 | |
| | Lymphocyte count decreased | 2 | unlikely |
| | Anemia | 2 | unlikely |
| | Platelet count decreased | 3 | unlikely |
| | ALAT | 0 | |
| | Fatigue | 1 | unlikely |
| | Hypotension | 1 | unlikely |
| | Abdominal Pain | 1 | unlikely |
| | Diarrhea | 1 | unlikely |
| | **Pain** | 1 | **unlikely** |
| | Fever | 0 | |

| **Treatment Day** | **Adverse event** | **Grade** | **Related to EKTOMUN** |
|---|---|---|---|
| 31.05.2012 | | | |
| **Dosis [µg/m2]** | White Blood cell decresed | 0 | unlikely |
| 100 | Neurophil count decreased | 0 | |
| | Lymphocyte count decreased | 2 | unlikely |
| | Anemia | 2 | unlikely |
| | Platelet count decreased | 3 | unlikely |
| | ALAT | 0 | |
| | Fatique | 1 | unlikely |
| | Hypotension | 0 | |
| | Abdominal Pain | 1 | unlikely |
| | Diarrhea | 1 | unlikely |
| | **Pain** | 1 | **unlikely** |
| | Fever | 1 | likely |
| | Flue like Symptoms | 2 | likely |
| | Malaise | 1 | likely |
| | Weight gain | 1 | likely |

**Table 2**

| **Treatment Day** | **Adverse event** | **Grade** | **Related to EKTOMUN** |
|---|---|---|---|
| 04.06.2012 | | | |
| **Dosis [µg/m2]** | White Blood cell decresed | 0 | unlikely |
| 150 | Neurophil count decreased | 0 | |
| | Lymphocyte count decreased | 3 | unlikely |
| | Anemia | 2 | unlikely |
| | Platelet count decreased | 3 | unlikely |
| | ALAT | 0 | |
| | Hypotension | 0 | |
| | Abdominal Pain | 1 | unlikely |
| | Diarrhea | 1 | unlikely |
| | **Pain** | 1 | **unlikely** |
| | Chills | 1 | likely |
| | Fever | 3 | likely |
| | Flue like Symptoms | 2 | likely |
| | Malaise | 1 | likely |
| | Weight gain | 0 | |
| | Fatigue | 2 | likely |
| | Somnolence | 1 | likely |
| | Hematuria | 1 | possible |

| **Treatment Day** | **Adverse event** | **Grade** | **Related to EKTOMUN** |
|---|---|---|---|
| 07.06.2012 | | | |
| **Dosis [µg/m2]** | White Blood cell decresed | 1 | unlikely |
| 200 | Neurophil count decreased | 0 | |
| | Lymphocyte count decreased | 3 | possible |
| | Anemia | 2 | unlikely |
| | Platelet count decreased | 3 | unlikely |
| | ALAT | 0 | |
| | Hypotension | 0 | |
| | Abdominal Pain | 0 | |
| | Diarrhea | 0 | |
| | **Pain** | **0** | |
| | Chills | 2 | likely |
| | Fever | 3 | likely |
| | Flue like Symptoms | 2 | likely |
| | Malaise | 1 | likely |
| | Weight gain | 0 | |
| | Fatigue | 1 | likely |
| | Somnolence | 1 | likely |
| | Hematuria | not evaluated | |

**Table 3**

| **Treatment day** | **Adverse event** | **Grade** | **Related to EKTOMUN** |
|---|---|---|---|
| 11.06.2012 | | | |
| **Dosis [µq/m2]** | White Blood cell decresed | 1 | unlikely |
| 200 | Neurophil count decreased | 0 | |
| | Lymphocyte count decreased | 3 | possible |
| | Anemia | 2 | unlikely |
| | Platelet count decreased | 3 | unlikely |
| | ALAT | 0 | |
| | Hypotension | 1 | possible |
| | Abdominal Pain | 0 | |
| | Diarrhea | 0 | |
| | **Pain** | **0** | |
| | Chills | 0 | |
| | Fever | 2 | likely |
| | Flue like Symptoms | 0 | |
| | Malaise | 1 | likely |
| | Weight gain | 0 | |
| | Fatigue | 1 | likely |
| | Somnolence | | likely |
| | Hematuria | 0 | |

| **Treatment Day** | **Adverse event** | **Grade** | **Related to EKTOMUN** |
|---|---|---|---|
| 14.06.2012 | | | |
| **Dosis [µq/m2]** | White Blood cell decresed | 0 | |
| 200 | Neurophil count decreased | 0 | |
| | Lymphocyte count decreased | | |
| | Anemia | 2 | unlikely |
| | Platelet count decreased | 3 | unlikely |
| | ALAT | 0 | |
| | Hypotension | 0 | |
| | Abdominal Pain | 0 | |
| | Diarrhea | 0 | |
| | **Pain** | **0** | |
| | Chills | 0 | |
| | Fever | 0 | |
| | Flue like Symptoms | 0 | |
| | Malaise | 0 | |
| | Weight gain | 0 | |
| | Fatigue | 0 | |
| | Somnolence | 0 | |
| | Hematuria | NE | |

### Summary and conclusion

The complement dependent cytotoxicity results demonstrate a significant better destruction of tumor cells by the parental anti-EpCAM antibody HO-3 than the bispecific antibody Catumaxomab. Whereas HO-3 showed complement dependent cytotoxicity over all antibody concentrations even at 50ng/ml, Catumaxomab was not able to lyse cells reproducibly and quantitatively at antibody concentrations < 500ng/ml.

Responsible for this observation can be the monovalent binding of the bispecific antibody to the tumor-associated antigen. Bivalent binding antibodies can bind with higher avidity to the tumor target and thus mediate efficient complement dependent tumor cell lysis also at lower antibody concentrations.

The following features of the presently disclosed bispecific antibody can be connected to further reducing or mitigating the complement binding associated nerve pain to an uncritical or non-existing level: the affinity of the presently disclosed antibody, preferably an antibody binding to GD2, displays a low to intermediate affinity to the tumor-associated antigen, preferably in a range of K_{D} = 10⁻⁵ ― 10⁻⁸ M , so that opsonization of target cells, e.g. GD2 positive tumor, or healthy tissue can only take place due to involved Fc-gamma receptor positive cells or T cells which increase the avidity of bound antibodies, e.g. anti-GD2 antibodies or anti-GD2 x anti-CD3 bispecific antibodies, to sufficient binding levels.

The reason for the low complement binding in the case of the presently disclosed antibody probably resides with the low affinity of the monovalent binding to the tumor-associated antigen e.g. GD2. That is, the presently disclosed antibodies bind first to the T cell and can only then bind to and destroy the tumor cells (e.g. GD2 positive tumor cells in the case of Ektomun) via the increased avidity of e.g. several hundreds of trAk molecules on the opsonized T cell, wherein complement may not play a role here, as the T cell expresses a number of complement-inhibiting antigens on its surface.

### References for the Background part

1. Zhang S, Cordon-Cardo C, Zhang HS, Reuter VE, Adluri S, Hamilton WB, Lloyd KO, Livingston PO: Selection of tumor antigens as targets for immune attack using immunohistochemistry: I: Focus on gangliosides. Int J Cancer 1997, 73:42―49.
2. Svennerholm L, Bostrom K, Fredman P, Jungbjer B, Lekman A, Mansson JE, Rynmark BM: Gangliosides and allied glycosphingolipids in human peripheral nerve and spinal cord. Biochim Biophys Acta 1994, 1214:115-123.
3. Hamilton WB, Helling F, Lloyd KO, Livingston PO: Ganglioside expression on human malignant melanoma assessed by quantitative immune thinlayer chromatography. Int J Cancer 1993, 53:566-573.
4. Mujoo K, Cheresh DA, Yang HM, Reisfeld RA: Disialoganglioside GD2 on human neuroblastoma cells: target antigen for monoclonal antibodymediated cytolysis and suppression of tumor growth. Cancer Res 1987, 47:1098-1104.
5. Grant SC, Kostakoglu L, Chris MG, Yeh SDJ, Larson SM, Finn RD, Oettgen HF, Cheung NKV: Targeting of small cell lung cancer using the anti-GD2 ganglioside monoclonal antibody 3F8: a pilot trial. Eur J Nucl Med 1996, 23:145-149.
6. Yoshida S, Fukumoto S, Kawaguchi H, Sato S, Ueda R, Furukawa K: Ganglioside G(D2) in small cell lung cancer cell lines: enhancement of cell proliferation and mediation of apoptosis. Cancer Res 2001, 61:4244―4252.
7. Cheresh DA, Rosenberg J, Mujoo K, Hirschowitz L, Reisfeld RA: Biosynthesis and expression of the disialoganglioside GD2, a relevant target antigen on small cell lung carcinoma for monoclonal antibody-mediated cytolysis. Cancer Res 1986, 46:5112-5118.
8. Lammie G, Cheung N, Gerald W, Rosenblum M, Cordoncardo C. Ganglioside gd expression in the human nervous-system and in neuroblastomas - an immunohistochemical study. Int J Oncol. (1993) 3:909-15. doi: 10.3892/ijo.3.5.909
9. Schulz G, Cheresh DA, Varki NM, Yu A, Staffileno LK, Reisfeld RA. Detection of ganglioside GD2 in tumor tissues and sera of neuroblastoma patients. Cancer Res. (1984) 44(12 Pt 1):5914-20.
10. Svennerholm L, Bostrom K, Fredman P, Jungbjer B, Lekman A, Mansson JE, et al. Gangliosides and allied glycosphingolipids in human peripheral nerve and spinal cord. Biochim Biophys Acta (1994) 1214:115-23. doi: 10.1016/0005-2760(94)90034-5
11. Mody R, Naranjo A, Van Ryn C, Yu AL, London WB, Shulkin BL, et al. Irinotecan-temozolomide with temsirolimus or dinutuximab in children with refractory or relapsed neuroblastoma (COG ANBL1221): an open-label, randomised, phase 2 trial. Lancet Oncol. (2017) 18:946-57. doi: 10.1016/S1470-2045(17)30355-8
12. Navid F, Sondel PM, Barfield R, Shulkin BL, Kaufman RA, Allay JA, et al. Phase I trial of a novel anti-GD2 monoclonal antibody, Hu14.18K322A, designed to decrease toxicity in children with refractory or recurrent neuroblastoma. J Clin Oncol. (2014) 32:1445-52. doi: 10.1200/JCO.2013.50.4423
13. Kushner BH, Kramer K, Modak S, Cheung NK. Successful multifold dose escalation of anti-GD2 monoclonal antibody 3F8 in patients with neuroblastoma: a phase I study. J Clin Oncol. (2011) 29:1168-74. doi: 10.1200/JCO.2010.28.3317
14. Osenga KL, Hank JA, Albertini MR, Gan J, Sternberg AG, Eickhoff J, et al. A phase I clinical trial of the hu14.18-IL2 (EMD 273063) as a treatment for children with refractory or recurrent neuroblastoma and melanoma: a study of the Children's Oncology Group. Clin Cancer Res. (2006) 12:1750-9. doi: 10.1158/1078-0432.CCR-05-2000
15. Isaacs JD, Greenwood J,Waldmann H. Therapy with monoclonal antibodies. II. The contribution of Fc gamma receptor binding and the influence of C(H)1 and C(H)3 domains on in vivo effector function. J Immunol. (1998) 161:3862-9.
16. Yu AL, Gilman AL, Ozkaynak MF, et al. Anti-GD2 antibody with GM-CSF, interleukin-2, and isotretinoin for neuroblastoma.(N Engl J Med. 2010;363:1324-1334).

## Claims

1. An intact whole IgG bispecific antibody, comprising the following properties:
a) binding to a T cell;
b) binding to a tumor-associated antigen on a tumor cell;
c) no or only marginal complement dependent cytotoxicity at antibody concentrations <500ng/ml resulting in the avoidance of nerve pain.

2. The intact bispecific antibody of claim 1, wherein the tumor-associated antigen is selected from a group consisting of tumor associated sphingolipids and sphingoid molecules, gangliosides or glucosphingolipids like e.g. GD2, GD3, GM1, GM2, GM3, fucosyl-GM1, globo-H, S1P, Cer, and Gg3, preferably the tumor-associated antigen is the neuroblastoma-associated antigen GD2.

3. The intact bispecific antibody of claim 1 or 2, wherein said intact bispecific antibody binds to the T cell through a T cell surface antigen, and wherein the T cell surface antigen is selected from a group consisting of CD2, CD3, CD4, CD8, CD28, CD40L and CD44, preferably the T cell surface antigen is CD3.

4. The intact bispecific antibody of any one of claims 1 to 3, wherein said antibody comprises an Fc- portion where the complement binding amino acids within the Fc-portion are modified by one of substitution, insertion and deletion.

5. A pharmaceutical composition comprising the intact bispecific antibody of any one of claims 1 to 4.

6. The pharmaceutical composition of claim 5, further comprising a pharmaceutically suitable carrier.

7. The intact bispecific antibody of any one of claims 1 to 4 for use as a medicament.

8. The intact bispecific antibody of any one of claims 1 to 4 for use in treatment of neuroblastoma or sarcoma, melanoma, glioma, small cell lung cancer, breast cancer, triple negative breast cancer, more preferably neuroblastoma, breast cancer or triple negative breast cancer.

9. The intact bispecific antibody for use of claim 7 or 8, wherein the antibody binds to cancer stem cell or cancer stem-like cells.

10. The intact bispecific antibody for use of any one of claims 7 to 9, wherein the antibody binds to breast cancer stem cell or breast cancer stem-like cells.

11. The intact bispecific antibody for use of any one of claims 7 to 10, wherein said antibody binds to GD2 expressed on the surface of tumor, cancer, cancer stem cell, or cancer stem-like cells.

12. The intact bispecific antibody for use of any one of claims 7 to 11, wherein said bispecific antibody is an anti-GD2 x anti-CD3 antibody.

13. The intact bispecific antibody for use of any one of claim 7 to 12, wherein said intact bispecific antibody is administered in an amount of 10 µg/m² body surface to 10 mg/m² body surface for one administration.

14. The intact bispecific antibody for use of any one of claims 7 to 13, wherein said bispecific antibody is administered for several times

15. The intact bispecific antibody for use of any one of claims 7 to 14, wherein said bispecific antibody is administered through intravenous injection, intraperitoneal injection, intramuscular injection, subcutaneous injection, or direct injection to the tumor.
